# EUROPEAN PATENT APPLICATION

(11) **EP 1 132 396 A1**
(43) Date of publication of application: **12.09.2001**
(21) Application number: 99972217.6
(22) Date of filing: 12.11.1999
(51) Int. Cl.: C07K 5/103, A61K 38/06, A61K 38/07, A61P 17/14, A61P 37/04, A61K 7/06

(54) **PHYSIOLOGICALLY ACTIVE PEPTIDES**

(30) Priority: 13.11.1998 JP 32333198; 03.03.1999 JP 5495499
(71) Applicant: KYOWA HAKKO KOGYO CO., LTD., Chiyoda-ku, Tokyo 100-8185 (JP)
(72) Inventor: YOSHIKAWA, Masaaki, Joyo-shi, Kyoto 610-0114 (JP); TAKAHATA, Kyoya, Okayama-shi, Okayama 700-0082 (JP); FURUKAWA, Tadayasu, Head Office, Chiyoda-ku, Tokyo100- 8185 (JP); TAKAHASHI, Tomoya, Tsukuba Research Laboratories, Tsukuba-shi, Ibaraki 305-0841 (JP)
(74) Representative: Casalonga, Axel
(86) International application number: JP9906329
(87) International publication number: WO0029425

(57) **Abstract**

The present invention provides peptides represented by the formula: R¹-Met-Ile-X-R² (wherein X represents Trp, Phe, Trp-Leu, Phe-Leu, Tyr-Leu, Ile-Leu or Leu-Leu; R¹ represents hydrogen or an amino-protecting group; and R² represents hydroxy or a carboxy-protecting group) or pharmaceutically acceptable salts thereof.

## Description

### Technical Field

The present invention relates to peptides having hair-growing activity, immune system-activating action, etc., and products containing the peptides, i.e., a hair-growing food, a hair-growing agent, an oral hair-growing agent and an immune system-activator.

### Background Art

It is known that a soybean protein-derived peptide represented by the formula: Met-Ile-Thr-Leu (MITL) and some other peptides having the sequence of MITL in the molecule exhibit immune system-activating action (e.g., phagocytosis-promoting activity, active oxygen production-promoting activity and the activity to promote tumor necrosis factor secretion) (Japanese Published Unexamined Patent Application Nos. 224093/95 and 73374/96) and anti-alopecia activity (Japanese Published Unexamined Patent Application No. 249535/97).

A known example of food intended for the purpose of hair-growing is that containing an extract of Gynostemma pentaphyllum Makino, that of leaves of Diospyros kaki Thunb. and that of sea tangle (Japanese Published Unexamined Patent Application No. 251866/85).

Also known is an oral anti-alopecia agent containing oolong tea extract as an active ingredient intended for the purpose of inhibition, improvement or prevention of alopecia caused as a side effect of an anti-tumor agent (Japanese Published Unexamined Patent Application No. 309840/97).

Further, finasteride, which is a Type II 5α-reductase inhibitor, is known as an oral hair-growing drug [Australasian Journal of Dermatology, 38, 20 (1997)]. However, as this drug acts on the metabolism of male sex hormone, great caution and lots of restrictions are needed on its use considering the possibility of side effects.

An object of the present invention is to provide peptides having hair-growing activity, immune system-activating action, etc., and products containing the peptides, i.e., a hair-growing food, a hair-growing agent, an oral hair-growing agent and an immune system-activator.

### Disclosure of the Invention

The present invention relates to peptides represented by the formula: R¹-Met-Ile-X-R² (wherein X represents Trp, Phe, Trp-Leu, Phe-Leu, Tyr-Leu, Ile-Leu or Leu-Leu; R¹ represents hydrogen or an amino-protecting group; and R² represents hydroxy or a carboxy-protecting group) (hereinafter referred to as MIX) or pharmaceutically acceptable salts thereof. In another embodiment, the present invention relates to the above MIX in which X is Trp, Trp-Leu, Phe-Leu, Tyr-Leu, Ile-Leu or Leu-Leu (hereinafter referred to as MIX') or pharmaceutically acceptable salts thereof. Preferred are peptides wherein R¹ is hydrogen and R² is hydroxy, or pharmaceutically acceptable salts thereof (hereinafter the peptide in which R¹ is hydrogen, R² is hydroxy and X is Trp is referred to as MIW; the peptide in which R¹ is hydrogen, R² is hydroxy and X is Phe is referred to as MIF; the peptide in which R¹ is hydrogen, R² is hydroxy and X is Trp-Leu is referred to as MIWL; the peptide in which R¹ is hydrogen, R² is hydroxy and X is Phe-Leu is referred to as MIFL; the peptide in which R¹ is hydrogen, R² is hydroxy and X is Tyr-Leu is referred to as MIYL; the peptide in which R¹ is hydrogen, R² is hydroxy and X is Ile-Leu is referred to as MIIL; and the peptide in which R¹ is hydrogen, R² is hydroxy and X is Leu-Leu is referred to as MILL).

The present invention also relates to peptides having the sequence Met-Ile-X (wherein X has the same significance as defined above) in the molecule (hereinafter MIX and the peptides having the sequence Met-Ile-X in the molecule are collectively referred to as MIXC) or pharmaceutically acceptable salts thereof. In another embodiment, the present invention relates to the above MIXC in which X is Trp, Trp-Leu, Phe-Leu, Tyr-Leu, Ile-Leu or Leu-Leu (hereinafter referred to as MIX'C) or pharmaceutically acceptable salts thereof.

The present invention provides a pharmaceutical comprising MIXC, MIX, MIX'C, MIX' or a pharmaceutically acceptable salt thereof.

The present invention also provides a hair-growing agent comprising, as an active ingredient, MIXC, MIX, MIX'C, MIX' or a pharmaceutically acceptable salt thereof.

The present invention also provides an oral hair-growing agent comprising, as an active ingredient, MIXC, MIX, MIX'C, MIX' or a pharmaceutically acceptable salt thereof.

The present invention also provides an immune system-activator comprising, as an active ingredient, MIXC, MIX, MIX'C, MIX' or a pharmaceutically acceptable salt thereof.

The present invention further provides a hair-growing food comprising, as an active ingredient, MIXC, MIX, MIX'C, MIX' or a pharmaceutically acceptable salt thereof.

Preferred are MIFL, MIYL, MIIL, MILL or pharmaceutically acceptable salts thereof, and a pharmaceutical, a hair-growing agent, an oral hair-growing agent, an immune system-activator and a hair-growing food containing any of them.

Examples of the amino-protecting group in the definition of the above formula are those described in Nobuo Izumiya, et al., Fundamentals and Experiments of Peptide Synthesis, Maruzen (1985). Preferred are lower alkanoyl, etc. Examples of the carboxy-protecting group are those described in Nobuo Izumiya, et al., Fundamentals and Experiments of Peptide Synthesis, Maruzen (1985). Preferred are lower alkoxy, amino, mono- or di-lower alkylamino, etc.

The lower alkyl moiety of the lower alkanoyl, the lower alkoxy and the mono- or di-lower alkylamino includes straight-chain or branched alkyl groups having 1-6 carbon atoms, such as methyl, ethyl, propyl, isopropyl, butyl, isobutyl, sec-butyl, tert-butyl, pentyl, isoamyl, neopentyl and hexyl.

The abbreviations for amino acids used herein are generally used in this field and have the following significances.
Met: L-Methionine
Ile: L-Isoleucine
Trp: L-Tryptophan
Leu: L-Leucine
Phe: L-Phenylalanine
Tyr: L-Tyrosine
Thr: L-Threonine

Examples of the pharmaceutically acceptable salts of the peptides of the present invention are inorganic acid addition salts such as hydrochloride, sulfate and phosphate, and organic acid addition salts such as acetate, maleate, fumarate, tartrate, citrate, lactate and methanesulfonate.

The process for producing the peptides of the present invention is described below.

The peptides of the present invention can be produced by conventional methods of peptide synthesis such as the solid-phase method and the liquid-phase method. For example, the peptides can be produced according to the methods described in Haruaki Yajima and Shunpei Yanagihara, The Japanese Biochemical Society ed., Lectures on Biochemical Experiments (I) : Chemistry of Protein, Vol. 4, Tokyo Kagaku Dojin (1977); Nobuo Izumiya, et al., Fundamentals and Experiments of Peptide Synthesis, Maruzen (1985), etc. or similar ones.

When the solid-phase method is employed, the peptides can be produced, for example, in the following manner.

p-Benzyloxybenzyl alcohol-type resin (polystyrene) as a support to obtain a peptide to which one α-amino-protected amino acid is bound by an ester bond is commercially available. It is preferred to protect the α-amino group of the amino acid to be used with a 9-fluorenylmethoxycarbonyl (Fmoc) group, and the indolyl group of tryptophan with a trityl (Trt) group. Condensation of protected amino acids is preferably carried out using a condensing agent such as dicyclohexylcarbodiimide (DCC) or benzotriazol-1-yloxytripyrrolidinophosphonium hexafluorophosphate (PyBOP) in the presence of N-hydroxybenzotriazole (HOBt). It is preferred to use N,N-dimethylformamide (DMF) as a solvent, and N-methylmorpholine for the removal of an Fmoc group. A crude synthetic peptide can be obtained by binding amino acids successively to synthesize a protected peptide on a resin and then treating the peptide on the resin with an acid such as trifluoroacetic acid (TFA) in the presence of a sulfur-containing compound such as thioanisole, ethanedithiol, ethyl methyl sulfide or thiophenol. In the usual condensation reaction, the protected amino acid, the condensing agent and HOBt are used in an amount of 1-15 equivalents based on the hydroxyl group of the resin or the first amino acid bound to the resin. The reaction is carried out at room temperature for 30 minutes to 5 hours. Release of the synthetic peptide from the resin is carried out by reaction at room temperature for 1-10 hours.

The obtained peptide can be purified by high performance liquid chromatography (HPLC) using a reversed-phase silica gel column, or column chromatography or thin layer chromatography such as partition, adsorption resins, silica gel, alumina, diatomaceous earth, magnesium silicate, ion-exchange resins or gel filtration.

The pharmaceutically acceptable salts of the peptides of the present invention can be obtained, for example, by dissolving the peptide in an aqueous solution of the corresponding acid and freeze-drying the resulting solution.

The peptides of the present invention and pharmaceutically acceptable salts thereof may be present in the form of adducts with water or various solvents, and these adducts are also within the scope of the present invention.

The physicochemical properties of the peptides of the present invention obtained by the above-described process are shown below.

### MIW

Property: White powder
Mass spectrum: m/z=489 (M+H)⁺
Water solubility: 1 mg/ml

### MIF

Property: White powder
Mass spectrum: m/z=410 (M+H)⁺
Water solubility: 30 mg/ml

### MIWL

Property: White powder
Mass spectrum: m/z=562 (M+H)⁺
Water solubility: 0.2 mg/ml

### MIFL

Property: White powder
Mass spectrum: m/z=523 (M+H)⁺
Water solubility: 0.2 mg/ml

### MIYL

Property: White powder
Mass spectrum: m/z=539 (M+H)⁺
Water solubility: 0.3 mg/ml

### MIIL

Property: White powder
Mass spectrum: m/z=489 (M+H)⁺
Water solubility: 0.2 mg/ml

### MILL

Property: White powder
Mass spectrum: m/z=489 (M+H)⁺
Water solubility: 0.2 mg/ml

The activities of the peptides of the present invention are shown below by test examples.

### Test Example 1 Cell Growth-Promoting Effect on Cultured Mouse Hair Follicle Cells

Mouse hair follicle cells were separated and cultured according to a modification of the method of Tanigaki, et al. [Archives of Dermatological Research, 284, 290-296 (1992)].

The skin on the back of a 4-days-old C3H mouse (Nippon Charles River Co., Ltd.) was cut off and treated with Eagle's MEM (Eagle's Minimum Essential Medium, Nissui Pharmaceutical Co., Ltd.) containing 500 units/ml Dispase (Godo Shusei Co., Ltd.) and 5% fetal calf serum (FCS) at 4°C for 16 hours. Then, the epidermis was stripped from the skin section, and the obtained dermis layer was treated with DMEM (Dulbecco's Modified Eagle Medium) containing 0.25% Collagenase N-2 (Nitta Gelatin Co., Ltd.) and 10% FCS at 37°C for one hour to obtain a dermis suspension. The dermis suspension was filtered through a 212-*µ*m nylon mesh (Nippon Rikagaku Kikai Co., Ltd.) and the filtrate was centrifuged at 1000 rpm for 5 minutes to obtain pellets containing hair follicle tissue. To the pellets was added calcium/magnesium-free PBS (Dulbecco's Phosphate-Buffered Saline) and the pellets were suspended therein using a pipette. The resulting suspension was allowed to stand for 15 minutes to precipitate the hair tissue. The same procedure as above (addition of calcium/magnesium-free PBS, preparation of a suspension using a pipette, and precipitation by standing for 15 minutes) was repeated three times using the obtained hair tissue. Then, the hair tissue was treated with an aqueous solution containing 0.1% ethylenediaminetetraacetic acid (EDTA) and 0.25% trypsin (Gibco) at 37°C for 5 minutes. To the resulting mixture was added DMEM containing 10% FCS to prepare a hair tissue cell suspension having a density of 3 x 10⁵ cells/ml.

This hair tissue cell suspension was put into wells of a 24-well collagen-coated plate (Iwaki Glass Co., Ltd.) in an amount of 1 ml/well, followed by culturing in 5% CO₂ at 37°C for 24 hours. After the completion of culturing, the medium was replaced by a medium prepared by adding to MCDB153 medium (Kyokuto Pharmaceutical Ind. Co., Ltd.) 5 mg/l bovine insulin (Sigma Chemical Co.), 5 *µ*g/l mouse epidermal growth factor (EGF) (Takara Shuzo Co., Ltd.), 40 mg/l bovine pituitary extract (Kyokuto Pharmaceutical Ind. Co., Ltd.), 10 mg/l human transferrin (Sigma Chemical Co.), 0.4 mg/l hydrocortisone (Sigma Chemical Co.), 0.63 *µ*g/l progesterone (Collaborative Research Co.), 14 mg/l O-phosphoethanolamine (Sigma Chemical Co.), 6.1 mg/l ethanolamine (Sigma Chemical Co.), 50 U/ml penicillin (Wako Pure Chemical Industries, Ltd.), 50 *µ*g/ml streptomycin (Wako Pure Chemical Industries, Ltd.) and a dimethyl sulfoxide (DMSO) solution containing a peptide of the present invention (added in an amount of 1/100 by volume based on the medium), followed by further culturing in 5% CO₂ at 37°C for 5 days. During the culturing, the medium was replaced with a fresh one every other day.

As a control, the cells were cultured in the same medium as above except that DMSO containing no peptide was added in an amount of 1/100 by volume based on the culture in place of DMSO containing the peptide.

The degree of cell growth was measured referring to the method using Neutral Red [Journal of Tissue Culture Method, 9, 1, 7-9 (1984)].

After the completion of culturing, the medium was sucked and the resulting cells were cultured in MCDB153 medium containing 50 mg/l Neutral Red (Sigma Chemical Co.) in 5% CO₂ at 37°C for 3 hours. After removal of the culture supernatant, the cultured cells were washed and immobilized with a 1% aqueous solution of formalin containing 1% calcium chloride for one minute. After washing and immobilization, the supernatant was removed, and a 50% aqueous solution of ethanol containing 1% acetic acid was added to the wells (0.4 ml/well = 2 cm²) to extract Neutral Red. The degree of cell growth was determined by measuring the absorbance of the extract at 540 nm.

The cell growth-promoting activity of the peptide of the present invention on hair follicle cells is shown in Table 1. The results are expressed in terms of the relative cell growth rate (%) based on the control.

**Table 1**

| Peptide | Concentration (*µ*M) | Relative cell growth rate (%) (based on the control) |
|---|---|---|
| MIW | 1 | 97 |
| | 3 | 143 |
| | 10 | 130 |
| | 30 | 123 |
| | 100 | 119 |

The physiologically active peptide of the present invention exhibited a significant cell growth-promoting effect on mouse hair follicle cells.

### Test Example 2 Effect on Hair Growth of Mouse

A test of the effect on hair growth of mice was carried out referring to the method of Ogawa, et al. [The Journal of Dermatology, 10, 45-54 (1983)].

Nine-weeks-old male C3H/HeSlc mice whose hair cycle was in the telogen (Japan SLC) were divided into groups each consisting of 4 or 5 mice. Hair on the back of each mouse was carefully shaven using electric hair clippers and an electric shaver. Then, the mice were fed with the feeds prepared in Reference Examples 1-4.

The control group was fed only with a powder feed containing no peptide of the present invention (CE-2, Clea Japan, Inc.)

On the 20th day after the start of the test, the skin on the back of each mouse was cut off and photographed. Using an image processor (Avionics Co., Spicca II), the percentage of the hair-grown area to the total area of the skin on the back was calculated. The rate of the increased hair-grown area (%) was obtained by subtracting the hair-growing rate of the control group from the hair-growing rate of the group fed with a powder feed containing a peptide of the present invention.

The results are shown in Table 2.

**Table 2**

| Peptide | Amount administered (addition to feed) | Rate of increased hair-grown area (%) |
|---|---|---|
| MIWL | 0.05% | 34.4 |
| MIW | 0.05% | 23.7 |
| MIW | 0.005% | 20.7 |
| MIF | 0.005% | 26.8 |

As shown in Table 2, it was confirmed that the feeding with the feed containing the physiologically active peptide of the present invention significantly promoted the growth of mouse hair follicles.

### Test Example 3 Measurement of Phagocytosis Activity

To human peripheral blood was added physiological saline containing phosphate buffer (PBS), and the resulting mixture was centrifuged at 1000 rpm for 5 minutes to wash blood cells. Then, the blood cells were suspended in PBS to prepare a suspension having a density of 4 x 10⁶ cells/ml. To 100 *µ*l of the suspension was added 10 *µ*l of a solution of a peptide in PBS, followed by incubation at 37°C for 10 minutes. Then, 10 *µ*l of a suspension of 4 x 10⁸/ml fluorescence-labeled latex beads opsonized with human peripheral blood was added thereto, and the resulting mixture was incubated for 5 minutes. To the mixture was added PBS containing ethylenediaminetetraacetic acid (EDTA) to stop the reaction. The resulting mixture was centrifuged to separate blood cells, and an ammonium chloride hemolyzing agent was added thereto for hemolysis to obtain leukocytes. After the obtained leukocytes were suspended in PBS containing EDTA, the resulting suspension was subjected to flow cytometry, and the number of cells which phagocytosed latex beads was calculated.

The results are shown in Table 3. The results are expressed in terms of the rate based on the control (containing only PBS).

**Table 3**

| Peptide | Concentration (*µ*M) | Phagocytosis activity |
|---|---|---|
| MIWL | 3 | 3.04 ± 0.50 |
| | 1 | 3.11 ± 0.07 |
| | 0.3 | 2.74 ± 0.25 |
| | 0.1 | 1.93 ± 0.18 |
| MIFL | 3 | 3.06 ± 0.25 |
| | 1 | 2.45 ± 0.17 |
| | 0.3 | 1.68 ± 0.08 |
| | 0.1 | 1.10 ± 0.09 |
| MIYL | 10 | 3.12 ± 0.32 |
| | 3 | 2.21 ± 0.25 |
| | 1 | 1.32 ± 0.15 |
| MIIL | 30 | 2.95 ± 0.20 |
| | 10 | 2.52 ± 0.18 |
| | 3 | 1.62 ± 0.08 |
| | 1 | 1.18 ± 0.07 |
| MILL | 30 | 2.97 ± 0.17 |
| | 10 | 2.05 ± 0.08 |
| | 3 | 1.28 ± 0.09 |
| MITL | 30 | 2.34 ± 0.46 |
| | 10 | 1.60 ± 0.06 |
| | 3 | 1.34 ± 0.05 |

The hair-growing agent of the present invention may be in any of the dose forms such as tablets, capsules, powders, pills, powders, fine granules, granules, syrups, troches and injections. The administration route for the agent is not specifically limited. Examples of suitable administration routes are oral administration, intravenous administration, intraperitoneal administration, subcutaneous administration, percutaneous administration and intramuscular administration. In the case, for example, of oral administration, a pure preparation, a purified product or a partially-purified product of MIXC can be administered as it is, or together with pharmaceutically acceptable excipients. Suitable excipients include saccharides such as sorbitol, lactose, glucose and lactose, dextrin, starch, inorganic substances such as calcium carbonate and calcium sulfate, crystalline cellulose, distilled water, sesame oil, corn oil, olive oil, soybean oil, cottonseed oil, and any other generally employed excipients. In preparing the agent, additives such as binders (e.g. polyvinyl alcohol, hydroxypropyl cellulose and gelatin), lubricants (e.g. magnesium stearate and talc), dispersing agents, suspending agents, emulsifiers, diluents, buffers, antioxidants and antibacterial agents may also be used.

The dose of MIXC will vary depending upon the condition of the disease, the age of the patient, etc. For instance, when the agent is orally administered to an adult, it is suitable to administer MIXC in an amount of 10-10000 mg, preferably 50-2000 mg, more preferably 100-500 mg per day.

The immune system-activator of the present invention may be in any of the dose forms such as tablets, powders, fine granules, granules, capsules, powders, pills, syrups, troches and injections. The administration route for the agent is not specifically limited. Examples of suitable administration routes are oral administration, intravenous administration, intraperitoneal administration, subcutaneous administration, percutaneous administration and intramuscular administration. In the case, for example, of oral administration, a pure preparation, a purified product or a partially-purified product of MIXC can be administered as it is, or in the form of tablets, powders, fine granules, granules, capsules, powders, pills, syrups, troches, etc. comprising pharmaceutically acceptable excipients. Suitable excipients include saccharides such as sorbitol, lactose, glucose, dextrin, starch and lactose, inorganic substances such as calcium carbonate and calcium sulfate, crystalline cellulose, distilled water, sesame oil, corn oil, olive oil, soybean oil, cottonseed oil, and any other generally employed excipients. In preparing the agent, additives such as binders (e.g. polyvinyl alcohol, hydroxypropyl cellulose and gelatin), lubricants (e.g. magnesium stearate and talc), dispersing agents, suspending agents, emulsifiers, diluents, buffers, antioxidants and antibacterial agents may also be used.

The dose of MIXC will vary depending upon the condition of the disease, the age of the patient, etc. For instance, when the agent is administered to an adult, it is suitable to administer MIXC in an amount of 10-10000 mg, preferably 50-2000 mg, more preferably 100-500 mg per day.

The food of the present invention can be produced, for example, by adding MIXC to food materials, specifically those which substantially do not contain MIXC in a conventional process for producing a food. In this case, MIXC can be added in the form of a pure preparation, a purified product, a partially-purified product, etc. in such an amount that the MIXC content in the food becomes 0.01% or more, preferably 0.01-4%, more preferably 0.05-1%.

The hair-growing food of the present invention may be in any of the forms such as tablets, capsules, powders, pills, jelly, beverages, frozen food, powder food, sheet-shaped food, bottled food, canned food and retort food, as well as the processed forms such as liquid food, pre-digested nutrient food and elemental diet. Such foods may comprise general materials employed in producing foods, such as proteins, saccharides, fats, trace elements, vitamins, emulsifiers and flavors, and can be produced according to conventional processes.

Examples of the foods are juice, soft drinks, tea, lactic acid beverages, fermented milk, ices, dairy products (e.g. butter, cheese, yogurt, processed milk and skim milk), meat products (e.g. ham, sausage and hamburger), fish products (e.g. steamed, baked or fried fish paste), egg products (e.g. fried or steamed foods made of beaten eggs), confectionery (e.g. cookies, jelly and snacks), bread, noodles, pickles, smoked fish and meat, dried fish, preserved foods boiled down with soy, salted foods, soup and seasonings.

### Best Modes for Carrying Out the Invention

### Example 1 Preparation of Hair-Growing Tablets

Tablets (300 mg/tablet) are prepared from the following ingredients according to a conventional method.

| | |
|---|---|
| MIW | 50 mg |
| Lactose | 190 mg |
| Corn starch | 30 mg |
| Synthetic aluminium silicate | 12 mg |
| Carboxymethyl cellulose calcium | 15 mg |
| Magnesium stearate | 3 mg |

### Example 2 Preparation of Hair-Growing Hard Capsules

Hard capsules (360 mg/capsule) are prepared from the following ingredients.

| | |
|---|---|
| MIWL | 50 mg |
| Lactose | 190 mg |
| Corn starch | 100 mg |
| Hydroxypropyl cellulose | 20 mg |

MIWL (50 mg) is mixed with 190 mg of lactose and 100 mg of corn starch, and an aqueous solution of hydroxypropyl cellulose (20 mg) is added thereto. The resulting mixture is kneaded and then granulated according to a conventional method using an extruding granulator. The obtained granules are packed in gelatin hard capsules.

### Example 3 Preparation of Hair-Growing Soft Capsules

Soft capsules (170 mg/capsule) are prepared from the following ingredients.

| | |
|---|---|
| MIFL | 50 mg |
| Soybean oil | 120 mg |

MIW (50 mg) is mixed with 120 mg of soybean oil. The resulting mixture is packed in soft capsules according to a conventional method using a rotary dies automatic molding machine.

### Example 4 Preparation of Hair-Growing Powder

A powder preparation (1000 mg/package) is prepared from the following ingredients according to a conventional method.

| | |
|---|---|
| MIYL | 50 mg |
| Lactose | 760 mg |
| Corn starch | 190 mg |

### Example 5 Preparation of Immune System-Activating Tablets

Tablets are prepared from the following ingredients according to a conventional method.

| | |
|---|---|
| MIIL | 10.0 g |
| Lactose | 90.0 g |
| Dry corn starch | 2.0 g |
| Talc | 1.8 g |
| Calcium stearate | 0.2 g |

### Example 6 Preparation of Hair-Growing Drink

A hair-growing drink is prepared from the following ingredients.

| | |
|---|---|
| MILL | 1.0 g |
| Sodium benzoate | 1.0 g |
| Fructose | 10.0 g |
| Flavor | proper amount |
| Pigment | proper amount |

Purified water is added to make a volume of 1000 ml.

### Reference Example 1 Preparation of a Feed Containing MIWL for Test Animals

MIWL (50 mg) was mixed with 100 g of a powder feed (CE-2, Clea Japan, Inc.) in a mortar to obtain a homogeneous mixture to be used as a test feed.

### Reference Example 2 Preparation of a Feed Containing MIW for Test Animals

MIW (50 mg) was mixed with 100 g of a powder feed (CE-2, Clea Japan, Inc.) in a mortar to obtain a homogeneous mixture to be used as a test feed.

### Reference Example 3 Preparation of a Feed Containing MIW for Test Animals

MIW (5 mg) was mixed with 100 g of a powder feed (CE-2, Clea Japan, Inc.) in a mortar to obtain a homogeneous mixture to be used as a test feed.

### Reference Example 4 Preparation ofa Feed Containing MIF for Test Animals

MIF (5 mg) was mixed with 100 g of a powder feed (CE-2, Clea Japan, Inc.) in a mortar to obtain a homogeneous mixture to be used as a test feed.

### Industrial Applicability

The present invention provides peptides having hair-growing activity, immune system-activating action, etc., and products containing the peptides, i.e., a hair-growing food, a hair-growing agent, an oral hair-growing agent and an immune system-activator.

### [Sequence Listing Free Text]

- SEQ ID NO: 1 -: Description of the artificial sequence: synthetic amino acid
- SEQ ID NO: 2 -: Description of the artificial sequence: synthetic amino acid
- SEQ ID NO: 3 -: Description of the artificial sequence: synthetic amino acid
- SEQ ID NO: 4 -: Description of the artificial sequence: synthetic amino acid
- SEQ ID NO: 5 -: Description of the artificial sequence: synthetic amino acid

## Claims

1. A peptide represented by the formula: R¹-Met-Ile-X-R² (wherein X represents Trp, Phe, Trp-Leu, Phe-Leu, Tyr-Leu, Ile-Leu or Leu-Leu; R¹ represents hydrogen or an amino-protecting group; and R² represents hydroxy or a carboxy-protecting group) or a pharmaceutically acceptable salt thereof.

2. The peptide according to claim 1, wherein X is Trp, Trp-Leu, Phe-Leu, Tyr-Leu, Ile-Leu or Leu-Leu, or a pharmaceutically acceptable salt thereof.

3. The peptide according to claim 1 or 2, wherein R¹ is hydrogen and R² is hydroxy, or a pharmaceutically acceptable salt thereof.

4. A pharmaceutical comprising the peptide according to any of claims 1-3 or a pharmaceutically acceptable salt thereof.

5. A hair-growing agent comprising, as an active ingredient, the peptide according to any of claims 1-3 or a pharmaceutically acceptable salt thereof.

6. An oral hair-growing agent comprising, as an active ingredient, the peptide according to any of claims 1-3 or a pharmaceutically acceptable salt thereof.

7. An immune system-activator comprising, as an active ingredient, the peptide according to any of claims 1-3 or a pharmaceutically acceptable salt thereof.

8. A hair-growing food comprising, as an active ingredient, the peptide according to any of claims 1-3 or a pharmaceutically acceptable salt thereof.


SEQUENCE LISTING

<110> KYOWA HAKKO KOGYO CO., LTD.

<120> PHYSIOLOGICALLY ACTIVE PEPTIDES

<130> 11166EP1

<140>
<141>

<160> 5

<170> PatentIn Ver. 2.0

<210> 1
<211> 4
<212> PRT
<213> Artificial Sequence

<220>
<223> Description of Artificial Sequence:Synthetic Amino Acid

<400> 1
Met Ile Trp Leu
  1

<210> 2
<211> 4
<212> PRT
<213> Artificial Sequence

<220>
<223> Description of Artificial Sequence:Synthetic Amino Acid

<400> 2
Met Ile Phe Leu
  1

<210> 3
<211> 4
<212> PRT
<213> Artificial Sequence

<220>
<223> Description of Artificial Sequence:Synthetic Amino Acid

<400> 3
Met Ile Tyr Leu
  1

<210> 4
<211> 4
<212> PRT
<213> Artificial Sequence

<220>
<223> Description of Artificial Sequence:Synthetic Amino Acid

<400> 4
Met Ile Ile Leu
  1

<210> 5
<211> 4
<212> PRT
<213> Artificial Sequence

<220>
<223> Description of Artificial Sequence:Synthetic Amino Acid

<400> 5
Met Ile Leu Leu
  1